**Europäisches Patentamt**

**European Patent Office**

**Office européen des brevets**

(11) Veröffentlichungsnummer: **0 006 998 B1**

(12) EUROPÄISCHE PATENTSCHRIFT

(45) Veröffentlichungstag der Patentschrift:
30.12.81

(51) Int. Cl.³: **G 01 N 33/78**

(21) Anmeldenummer: **79101785.8**

(22) Anmeldetag: **06.06.79**

(54) Verfahren zur Bestimmung des Thyroxin-bindungsindex im Serum und Testkit zur Durchführung des Verfahrens.

(30) Priorität: **12.06.78 DE 2825650**

(43) Veröffentlichungstag der Anmeldung:
**23.01.80 Patentblatt 80/2**

(45) Bekanntmachung des Hinweises auf die Patenterteilung:
**30.12.81 Patentblatt 81/52**

(84) Benannte Vertragsstaaten:
**BE CH DE FR GB IT LU NL SE**

(56) Entgegenhaltungen:
**DE-A-2 719 836**
**DE-A-2 736 527**
**US-A-4 043 872**

(73) Patentinhaber: **BOEHRINGER MANNHEIM GMBH,
Sandhofer Strasse 112-132 Postfach 31 01 20,
D-6800 Mannheim 31-Waldhof (DE)**

(72) Erfinder: **Kleinhammer, Gerd, Dr., Heinrich Vogl
Strasse 1, D-8132 Tutzing (DE)**
Erfinder: **Deutsch, Gerlinde, Lindenberg 15,
D-8134 Pöcking (DE)**
Erfinder: **Linke, Hans-Ralf, Dr., Andechser Strasse 7,
D-8919 Raisting (DE)**
Erfinder: **Stähler, Fritz, Dr., Heimgartenstrasse 4,
D-8132 Tutzing (DE)**
Erfinder: **Gruber, Wolfgang, Dr., Am Oberanger 7,
D-8132 Tutzing-Unterzeismering (DE)**

(74) Vertreter: **Weickmann, Heinrich, Dipl.-Ing Patentanwälte
Dipl.Ing.H.Weickmann, Dipl.Phys.Dr.K.Fincke
Dipl.Ing.F.A.Weickmann Dipl.Chem.B.Huber,
Dr.-Ing.H.Liska Möhlstrasse 22, D-8000 München 86 (DE)**

# Verfahren zur Bestimmung des Thyroxin-bindungsindex im Serum und Testkit zur Durchführung des Verfahrens

Die Schilddrüsendiagnostik hat im Rahmen der Klinischen Chemie wegen der weiten Verbreitung von Schilddrüsenuntersuchungen besondere Bedeutung. Die Basis-in-vitro-Schilddrüsendiagnostik umfasst dabei die Ermittlung des gesamten im Serum vorhandenen Thyroxins (Gesamt-$T_4$) sowie die Bestimmung der Konzentration des thyroxinbindenden Globulins (TGB), welche mit Hilfe der sogenannten Trijodthyroninaufnahme ($T_3$-uptake) oder Thyroxinbindungsindextests (TBI-Tests) erfolgt.

Mit dem erwähnten $T_3$-uptake-test wird die Restbindekapazität (RBK) von Trägerproteinen für Schilddrüsenhormone im Serum bestimmt. Bei diesen Proteinen handelt es sich um das thyroxinbindende Globulin (TBG), an dem etwa 60% der gesamten vorhandenen $T_4$-Menge gebunden sind sowie um das thyroxinbindende Präalbumin (TBPA) und Albumin. Bei dieser Restbindekapazität handelt es sich im wesentlichen somit um die Bindung der Schilddrüsenhormone an das TBG. So sind im Fall einer Hyperthyreose mit z.B. erhöhtem $T_4$-Gehalt, mehr, bei der Hypothyreose weniger Bindungsstellen der Trägerproteine mit den Schilddrüsenhormonen $T_4$ und $T_3$ besetzt.

Bei einem bekannten Verfahren zur TBI-Bestimmung versetzt man eine bestimmte Menge von radioaktivem $T_3$ mit der Serumprobe und einem Anionenaustauscher. Die freien Bindungsstellen des Trägerproteins und der Anionenaustauscher konkurrieren um das radioaktive $T_3$. Je mehr unbesetzte Bindungsstellen an den Trägerproteinen vorhanden sind, desto mehr radioaktives $T_3$ wird dort gebunden, der Rest geht an den Ionenaustauscher. Die Auswertung erfolgt, indem man entweder die Radioaktivität am Ionenaustauscher oder in der Lösung misst. Das Verhältnis der Radioaktivität in der Lösung, die derjenigen Menge an radioaktivem $T_3$ entspricht, die an die Trägerproteine des zu untersuchenden Serums gebunden ist, zur Radioaktivität in der Lösung einer Standardprobe, multipliziert mit einem für den Standard spezifischen Faktor ergibt den sogenannten Thyroxinbindungsindex TBI.

Ein Nachteil dieses bekannten Verfahrens liegt in der Notwendigkeit, radioaktive Substanzen zu verwenden. Hierdurch werden einerseits teure und komplizierte Messvorrichtungen benötigt und andererseits ist die Handhabung der radioaktiven Reagentien durch gesetzliche Auflagen wegen der potentiellen Gesundheitsschädlichkeit dieser Substanzen erschwert.

Der Erfindung liegt daher die Aufgabe zugrunde, ein Verfahren zur TBI-Bestimmung zu schaffen, welches diese Nachteile nicht aufweist und es gestattet, mit einfachen, in jedem Laboratorium vorhandenen Messgeräten eine zuverlässige Bestimmung des TBI-Index für die Schilddrüsendiagnostik vorzunehmen.

Gelöst wird diese Aufgabe durch ein Verfahren zur Bestimmung des Thyroxinbindungsindex im Serum, welches dadurch gekennzeichnet ist, dass die zu untersuchende Serumprobe mit einer bestimmten Menge Thyroxin und einer bestimmten Menge eines kovalent an Thyroxin gebundenen, bestimmbaren Enzyms versetzt, die erhaltene Lösung mit in fester Phase vorliegenden Antithyroxin-antikörpern kontaktiert, die flüssige von der festen Phase getrennt und die Aktivität des eingesetzten bestimmbaren Enzyms in einer der Phasen gemessen wird.

Die Erfindung geht aus von der Überlegung, dass ein Ersatz der radioaktiven Markierung beim bekannten $T_3$-uptake-test durch eine Enzymmarkierung unter Verwendung eines leicht bestimmbaren Enzyms als Marker die gestellte Aufgabe lösen konnte. Die durchgeführten Versuche zeigten jedoch, dass es nicht möglich ist, bei dem bekannten $T_3$-uptake-test radioaktiv markiertes $T_3$ ($T_3$-$J_{125}$) durch enzymmarkiertes $T_3$ oder $T_4$ (welches vorzuziehen wäre) zu ersetzen. Es stellte sich nämlich heraus, dass enzymmarkiertes $T_3$ oder $T_4$ von den bindefähigen Stellen der Serumproteine nicht gebunden wird. Es ist anzunehmen, dass dies auf den voluminösen Enzymrest zurückzuführen ist, der sterisch die Bindung hindert.

Überraschenderweise wurde jedoch festgestellt, dass enzymmarkiertes $T_4$ mit nichtenzymmarkiertem $T_4$ um Anti-$T_4$-antikörper, die in der festen Phase vorliegen, zu konkurrieren vermögen. Beim erfindungsgemässen Verfahren konkurrieren daher einerseits die bindefähigen Stellen der Serumproteine und die der unlöslichen Anti-$T_4$-antikörper um das $T_4$, wobei um so mehr $T_4$ an den Anti-$T_4$-antikörper gebunden wird, je weniger freie Bindungsstellen im Serum vorhanden sind. Gleichzeitig konkurriert enzymmarkiertes $T_4$ mit nicht markiertem $T_4$ um den Anti-$T_4$-antikörper. Es wird daher um so mehr enzymmarkiertes $T_4$ an die feste Phase gebunden, je grösser die Thyroxinbindekapazität des Serums und damit der Thyroxinbindungsindex TBI ist. Die gemessene Enzymaktivität in der fesen Phase stellt daher ein direktes Mass für die Grösse des TBI dar. Es kann aber auch die Aktivität in der flüssigen Phase bestimmt werden.

Als Markierungsenzym kann im Rahmen des erfindungsgemässen Verfahrens jedes sich ohne weiteres bestimmen lassende Enzym verwendet werden, welches ohne Verlust seiner enzymatischen Aktivität an Thyroxin gebunden werden kann. Bevorzugt werden im Rahmen der Erfindung solche Markierungsenzyme verwendet, deren Aktivität sich leicht durch einen optischen Test bestimmen lässt, insbesondere durch eine Farbreaktion im sichtbaren Licht oder durch Änderung der NADH-Konzentration. Typische Beispiele für geeignete Markierungsenzyme sind Peroxidase (POD), Glucoseoxidase, $\alpha$- und $\beta$-Galactoxidase, Glucoamylase, Invertase und alkalische Phosphatase.

Für alle oben genannten Enzyme sind einfache und rasche Bestimmungsmethoden bekannt, wozu beispielsweise auf H.U. Bergmeyer «Methoden der enzymatischen Analyse», Verlag Chemie, 3. Auflage 1974, verwiesen wird. Der in fester Phase vorliegende Anti-$T_4$-antikörper kann trägerfrei vorliegen, z.B. durch Vernetzung mit polyfunktionellen Reagentien, wie Dialdehyden, Dioxiden und dergleichen unlöslich gemacht. Vorzugsweise ist der Antikörper jedoch an ein festes Trägermaterial gebunden. Der feste Träger für den Anti-$T_4$-antikörper kann aus jedem gegenüber den im Test verwendeten Substanzen inerten Material bestehen, an welches der erwähnte Antikörper gebunden werden kann. Es wurde gefunden, dass bei zahlreichen Materialien der Anti-$T_4$-antikörper durch Adsorptivkräfte ausreichend fest an die Oberfläche des Trägermaterials g ···den werden kann. Wahlweise ist es aber auch möglich, den Antikörper nach den üblichen Methoden der Fixierung biologisch aktiver Proteine an feste Trägermaterialien, z.B. über kovalente Bindungen, zu fixieren. Typische Beispiele hierfür sind die Aktivierung der Trägeroberfläche nach der Brom-cyan-methode, durch Proteincopolymerisation, oberflächliche Vernetzung des Antikörpers auf dem Träger durch polyfunktionelle Vernetzungsmittel, wie Glutardialdehyd und dergleichen. Diese Methoden sind dem Fachmann bekannt.

Bevorzugt werden im Rahmen der Erfindung Behälter, wie Reagenzgläschen, verwendet, deren Innenwand mit dem Antikörper beschichtet ist. Typische Beispiele für geeignete Behältermaterialien sind Polystyrol, Styrolacrylnitril-copolymerisate sowie andere Kunststoffe und Glas. Bei den Kunststoffen auf Styrolbasis genügt es, für eine ausreichende Beschichtung lediglich eine Antikörperlösung einige Zeit in dem Behälter aufzubewahren.

Die obigen Ausführungen gelten in analoger Weise für Trägermaterialien, welche nicht in Form von Behältern vorliegen, sondern beispielsweise aus Teilchen bestehen, die sich für Säulenfüllungen eignen.

Anti-$T_4$-antikörper werden nach den üblichen Methoden der Gewinnung von Antikörpern gegen Hormone hergestellt. Bevorzugt wird die Verwendung eines Konjugats von $T_4$ an einem geeigneten Trägerprotein als Immunogen verwendet. Als besonders geeignet aus der grossen Zahl der dem Fachmann geläufigen Trägerproteine erwies sich Rinderserumalbumin (RSA). Die Trägerproteine werden chemisch mit dem $T_4$ gekuppelt, beispielsweise mit Glutardialdehyd in schwach alkalischem Medium und anschliessende Reduktion mit Natriumborhydrid. Eine andere geeignete Kupplungsmethode ist Umsetzung mit Morpholino-dicyclohexyl-carbodiimid. Geeignete Versuchstiere für die Applikation des so erhaltenen $T_4$-Immunogens sind beispielsweise Kaninchen oder Schafe. Auch andere Tierarten sind geeignet.

Die Herstellung des im Rahmen der Erfindung bevorzugten, mit Peroxidase markierten $T_4$ erfolgt zweckmässig durch Umsetzung von 6-Butyloxy-carbonyl-thyroxin-N-hydroxy-succinimid mit POD und chromatographische Aufreinigung des erhaltenen Produkts. In gleicher Weise lassen sich die meisten anderen in Betracht kommenden Enzyme unter Verwendung auch anderer bekannter proteinchemischer Verfahren binden.

Wie erwähnt wird im Rahmen der Erfindung eine bestimmte Menge $T_4$ zugesetzt. Es wurde gefunden, dass diese zweckmässigerweise zwischen 100 und 500 ng $T_4$/ml Serum beträgt. Es können jedoch auch grössere oder kleinere Mengen angewendet werden. Der oben genannte Mengenbereich erfasst jedoch alle praktisch vorkommenden Serumzusammensetzungen und reicht gleichermassen für hyperthyroide wie für hypothyroide Seren aus. Die Menge des zugesetzten enzymmarkierten $T_4$ hängt ab von der Art des jeweils verwendeten Enzyms, insbesondere von der spezifischen Aktivität dieses Enzyms, die sich noch leicht bestimmen lässt. Im Falle der bevorzugten Markierung mit POD wird zweckmässigerweise eine solche Menge eingesetzt, dass sich im eigentlichen Testgemisch etwa 1 bis etwa 200 mU POD/ml befinden.

Das Reaktionsgemisch von Serum, Puffer, $T_4$ und enzymmarkiertem $T_4$ wird mit dem unlöslichen, insbesondere trägergebundenen Anti-$T_4$-antikörper ausreichend lange kontaktiert, um eine konstante Bindungsrate des markierten $T_4$ am Antikörper zu erhalten. Die Dauer hängt in gewissem Umfang von den Bedingungen hinsichtlich pH-Wert, Temperatur und Konzentrationen ab. Im allgemeinen reicht eine Einwirkungszeit von etwa ½ Stunde bis etwa 2 Stunden aus. Nach Beendigung der Inkubation wird die Lösung vom Träger abgetrennt, beispielsweise durch Ausgiessen aus dem Behälter, der Träger wird mit Wasser gewaschen und anschliessend die Bestimmung der trägergebundenen Enzymaktivität durchgeführt. Im Falle der bevorzugten Ausführungsform unter Verwendung eines behälterförmigen Trägers und von POD als Markierungsenzym wird beispielsweise $H_2O_2$ und ABTS® (2,2'-Azino-di [3-äthylbenzthiazolinsulfonat (6)]) in Pufferlösung zugesetzt und die Extinktionsdifferenz bei einer Messwellenlänge von 405 nm bestimmt.

Ein weiterer Gegenstand der Erfindung ist ein Testkit zur Durchführung des erfindungsgemässen Verfahrens. Er besteht im wesentlichen aus Thyroxin, enzymmarkiertem Thyroxin, Anti-thyroxin-antikörper als unlöslicher Feststoff, Puffersubstanz und einem Reagens zur Bestimmung der Enzymaktivität. Geeignete Puffer sind solche, mit denen ein pH-Wertbereich im Bereich von 7,5 bis etwa 9,3 eingestellt werden kann. Bevorzugt wird ein pH-Wert zwischen 8,0 und 9,0. Typische Beispiele für im angegebenen Bereich verwendbare Puffer sind Phosphatpuffer, Tris-puffer, Boraxpuffer, Barbitalpuffer.

Bevorzugt wird 0,05 bis 0,5 M Barbitalpuffer.

Der erfindungsgemässe Testkit kann zusätzlich noch übliche Stabilisierungsmittel, wie Rinderserumalbumin, Kohlehydrate, Glycerin enthalten.

Vorzugsweise enthält der erfindungsgemässe

Testkit als enzymmarkiertes $T_4$ Thyroxin-peroxidase. Das Reagens zur Bestimmung der Enzymaktivität kann dann in diesem bevorzugten Fall aus $H_2O_2$ oder einer $H_2O_2$ liefernden Verbindung, wie Natriumperborat oder Perhydrit sowie 2,2'-Azino-di[3-äthylbenzthaizolinsulfonat(6)], üblicherweise als ABTS® bezeichnet, sowie einem geeigneten Puffer hierfür bestehen. Typisches Beispiel für einen geeigneten Puffer ist Phosphat-Citrat-Puffer, pH 4,5 bis 6,0. Ein anderes für diese Ausführungsform geeignetes Reagens besteht aus Phosphatpuffer, pH 7,0, Guayakol und Wasserstoffperoxid.

In einer speziellen Ausführungsform des bevorzugten Testkits der Erfindung enthält dieser:

| | |
|---|---|
| Thyroxin | 100–400 ng/ml |
| Thyroxin-POD | 5–100 mU/ml |
| Barbitalpuffer | 0,1–0,2 M, pH 8,6 |
| Rinderserumalbumin | 0,2% |
| Antithyroxinantikörper (berechnet ohne Träger) | 1–0,05 µg/ml |
| $H_2O_2$ | 0,5–5 mM/l |
| ABTS | 5–50 mM/l |
| Phosphat-citratpuffer, pH 5,0 | 0,1–0,2 M |

Zur Herstellung des enzymmarkierten Thyroxins wird vorzugsweise ausgegangen von t-Butyloxycarbonyl-thyroxin-N-hydroxysuccinimid und die Umsetzung erfolgt in Puffer/Dimethylformamid(DMF)-lösung 1:1. Als Puffer wird der für das jeweils verwendete Enzym bestgeeignete Puffer verwendet. Zur Aufreinigung der Konjugate hat sich Phenylbutylaminsepharose bewährt.

Durch die Erfindung wird ein einfaches und mit üblichen Laborgeräten durchführbares Verfahren zur Bestimmung des Thyroxinbindungsindex des Serums geschaffen, dessen Genauigkeit der der bekannten Methode unter Verwendung von radioaktiver Markierung entspricht, jedoch deren Nachteile nicht aufweist.

Die folgenden Beispiele erläutern die Erfindung weiter.

Beispiel 1
A. Herstellung von Anti-thyroxin-antikörpern

Thyroxin wird in wässriger Lösung von pH 10 durch Zusatz von 1,9 Gew.-% Glutardialdehyd an Rinderserumalbumin gekuppelt. Dann wird mit überschüssigem Natriumborhydrid die Schiff-Basenbindung reduziert und das $T_4$-Immunogen durch Chromatographie gereinigt. Das erhaltene Produkt wird dialysiert und dann den Versuchstieren appliziert.

B. Herstellung von $T_4$-POD.

POD wird mit 10-fach molarem Überschuss an t-Butyloxycarbonyl-thyroxin-N-hydroxysuccinimid in Dimethylformamid/Phosphatpuffer, pH 8,5, 1:1 umgesetzt. Dann wird das DMF abdialysiert und die wässrige Lösung über Phenylbutylamin-sepharose gegeben. Die Säule wird mit Tris-NaCl-Puffer gewaschen und dann mit 1M NaCl enthaltendem Äthylenglykol/Wasser-ge-

misch 1:1 eluiert. Das Eluat wird 2 Stunden mit 0,5 M Hydroxylamin gerührt, anschliessend dialysiert gegen Phosphatpuffer. Die erhaltene Lösung wird mit Rinderserumalbumin (RSA) versetzt und lyophilisiert.

C. Herstellung von trägergebundenen Anti-$T_4$-antikörpern.

Aus den immunisierten Versuchstieren wird in bekannter Weise Anti-$T_4$-antiserum gewonnen und durch Ammonsulfat gefällt. Die Fällung wird mit 0,04 M Phosphatpuffer 1:6000 aufgenommen. 1,5 ml der so erhaltenen Lösung werden über Nacht in Reagenzgläschen aus Styrol-acrylnitrilmischpolymerisat (Luran) stehen gelassen, dann ausgesaugt und mit physiologischer Kochsalzlösung, die 1% RSA enthält, gewaschen und danach getrocknet.

D. Durchführung der Bestimmung.

In die nach C. erhaltenen Anti-$T_4$-antikörperbeschichteten Reagenzgläschen werden 10 µl Serum und anschliessend 1 ml eines Reagens pipettiert, welches 280 ng $T_4$/ml und 10 mU/ml $T_4$-POD in 0,12 M Barbitalpuffer, pH 8,6, und 0,2% Rinderserumalbumin enthält. Dann wird 2 Stunden bei Raumtemperatur stehen gelassen. Danach werden die Gläschen durch Aussaugen entleert und das POD-Reagens eingefüllt. Letzteres bestand aus 1,47 mM/l $H_2O_2$ und 14 mM/l ABTS in 0,2 M Phosphatcitratpuffer, pH 5,0. Die auftretende Farbänderung wurde bei 405 nm gemessen.

Zur Auswertung wird mit 2 Standards unterschiedlicher $T_4$-Bindekapazität, die in gleicher Weise wie die Proben behandelt werden, eine Eichgerade erstellt, indem die Reziprokwerte der für diese Standards gemessenen Extinktionen aufgetragen werden entweder gegen die von den Standards gebundenen $T_4$-Mengen oder direkt gegen die zuvor zu ermittelnden TBI-Werte dieser Standards.

E. Anwendung der Methode auf die Untersuchung von Humanseren.

83 Humanserumproben wurden vergleichend mit dem erfindungsgemässen und einem bereits im Handel befindlichen $T_3$-$J_{125}$ einsetzenden Verfahren untersucht. Klassifiziert man die untersuchten Seren aufgrund der jeweils erhaltenen TBI-Werte anhand des für die betreffende Methode gültigen Normalbereichs in solche mit verminderter, normaler und erhöhter $T_4$-Bindungskapazität, so zeigt sich, dass zwischen beiden Methoden eine befriedigende Übereinstimmung resultiert (siehe Tabelle 1).

Tabelle 1

| A / B | 1 | 2 | 3 |
|---|---|---|---|
| 1 | 28 | 1 | |
| 2 | 1 | 28 | |
| 3 | | 3 | 22 |

1) verminderte
2) normale ⎫
3) erhöhte ⎬ $T_4$-Bindungskapazität

7 | 0 006 998 | 8

Tab. 1: Vergleich des erfindungsgemässen Verfahrens zur Ermittlung des TBI (A) mit einem üblichen TBI-Radio-Assay (B) unter Verwendung von Humanserumproben (n = 83)

## Patentansprüche

1. Verfahren zur Bestimmung des Thyroxinbindungsindex im Serum, dadurch gekennzeichnet, dass die zu untersuchende Serumprobe mit einer bestimmten Menge Thyroxin und einer bestimmten Menge eines kovalent an Thyroxin gebundenen bestimmbaren Enzyms versetzt, die erhaltene Lösung mit in fester Phase vorliegenden Antithyroxin-antikörpern kontaktiert, die flüssige von der festen Phase abgetrennt und die Aktivität des eingesetzten bestimmbaren Enzyms in einer der Phasen gemessen wird.

2. Verfahren nach Anspruch 1, dadurch gekennzeichnet, dass als bestimmbares Enzym Peroxidase verwendet wird.

3. Verfahren nach Anspruch 1, dadurch gekennzeichnet, dass der Anti-thyroxin-antikörper in trägergebundener Form verwendet wird.

4. Verfahren nach Anspruch 3, dadurch gekennzeichnet, dass als fester Träger ein Behälter verwendet wird, dessen Innenwand mit dem Antikörper beschichtet ist.

5. Verfahren nach einem der Ansprüche 1 bis 4, dadurch gekennzeichnet, dass man 100 bis 500 ng Thyroxin/ml Serum einsetzt.

6. Verfahren nach einem der Ansprüche 1 bis 5, dadurch gekennzeichnet, dass man 1 bis 200 mU/ml thyroxingebundene Peroxidase einsetzt.

7. Testkit zur Durchführung des Verfahrens nach einem der Ansprüche 1 bis 6, dadurch gekennzeichnet, dass er Thyroxin, enzymmarkiertes Thyroxin, Antithyroxin-antikörper an Feststoff gebunden, Puffer, Reagens zur Bestimmung der Enzymaktivität und gegebenenfalls Stabilisierungsmittel und immunreaktionsfördernde Zusätze enthält.

8. Testkit nach Anspruch 7, dadurch gekennzeichnet, dass er

| | |
|---|---|
| Thyroxin | 100–400 ng/ml |
| Thyroxin-POD | 5–100 mU/ml |
| Barbitalpuffer | 0,1–0,2 M, pH 8,6 |
| Rinderserumalbumin | 0,2% |
| Antithyroxin-antikörper (berechnet ohne Träger) | 1–0,05 µg/ml |
| $H_2O_2$ | 0,5–5 mM/l |
| ABTS | 5–50 mM/l |
| Phosphat-citratpuffer, pH 5,0 | 0,1–0,2 M |

enthält oder daraus besteht.

## Claims

1. Process for the determination of the thyroxine bonding index in serum, whereby the serum sample to be examined is treated with a certain amount of a covalent determinable enzyme linked to thyroxine, the solution thus obtained is contacted by antithyroxine antibodies present in solid phase, the liquid phase is then separated from the solid phase and the activity of the determinable enzyme added is measured in one of the phases.

2. Process according to claim 1, characterized in that peroxidase is used as the determinable enzyme.

3. Process according to claim 1, whereby the anti-thyroxine antibody is used in carrier-bonded form.

4. Process according to claim 3, whereby a container is used as the solid carrier, the inner wall of which is coated with said antibody.

5. Process according to one of claims 1 to 4, whereby 100 to 500 ng thyroxine/ml of serum is added.

6. Process according to one of the claims 1 to 5, whereby 1 to 200 mU/ml of thyroxine-bonded peroxidase is added.

7. Testkit for the conduct of the process according to one of the claims 1 to 6 above, whereby it contains thyroxine, enzyme-marked thyroxine, antithyroxine antibodies linked to a solid, buffer, reagent for the determination of the enzyme activity and optionally stabilizing agents and additives promoting immune response.

8. A testkit according to claim 7 above whereby it contains or consists of:

| | |
|---|---|
| thyroxine | 100–400 ng/ml |
| thyroxine-POD | 5–100 mU/ml |
| barbital buffer | 0.1–0.2 M, pH 8.6 |
| beef serum albumin | 0.2% |
| antithyroxine antibodies (calculated without carrier) | 1–0.05 µg/ml |
| $H_2O_2$ | 0.5–5 mM/l |
| ABTS | 5–50 mM/l |
| phosphate-citrate buffer, pH 5.0 | 0.1–0.2 M |

## Revendications

1. Procédé pour le dosage de l'indice de liaison de la thyroxine dans le sérum, caractérisé en ce que l'échantillon de sérum à l'étude est additionné d'une quantité déterminée de thyroxine et d'une quantité déterminée d'une enzyme dosable liée de façon covalente à de la thyroxine, la solution obtenue est mise en contact avec des anticorps antithyroxine se présentant en phase solide, la phase liquide est séparée de la phase solide et l'activité de l'enzyme dosable mise en œuvre est mesurée dans l'une des phases.

2. Procédé selon la revendication 1, caractérisé en ce qu'on utilise de la peroxydase en tant qu'enzyme dosable.

3. Procédé selon la revendication 1, caractérisé en ce que l'anticorps antithyroxine est utilisé sous forme liée à un support.

4. Procédé selon la revendication 3, caractérisé en ce qu'on utilise en tant que support solide un récipient dont la paroi intérieure est revêtue avec l'anticorps.

5. Procédé selon l'une des revendications 1 à 4, caractérisé en ce qu'on met en œuvre 100 à 500 ng de thyroxine/ml de sérum.

6. Procédé selon l'une des revendications 1 à 5, caractérisé en ce qu'on met en œuvre 1 à 200 mU/ml de peroxydase liée à de la thyroxine.

7. Nécessaire pour essais (testkit) pour la mise en œuvre du procédé selon l'une des revendications 1 à 6, caractérisé en ce qu'il contient de la thyroxine, de la thyroxine marquée à l'enzyme, de l'anticorps antithyroxine lié à une matière solide, du tampon, du réactif pour le dosage de l'activité enzymatique et éventuellement des agents stabilisants et des additifs favorisant les réactions immunoloiques.

8. Nécessaire pour essais selon la revendification 7, caractérisé en ce qu'il contient ou est constitué de:

| | |
|---|---|
| thyroxine | 100–400 ng/ml |
| thyroxine-POD | 5–100 mU/ml |
| tampon au barbital | 0,1–0,2 M, pH 8,6 |
| Albumine du sérum de bovins | 0,2% |
| anticorps antithyroxine (calculé sans support) | 1–0,05 µg/ml |
| $H_2O_2$ | 0,5–5 mM/l |
| ABTS | 5–50 mM/l |
| tampon au phosphate-citrate, pH 5,0 | 0,1–0,2 M |